(19) Europäisches Patentamt
European Patent Office
Office européen des brevets

(11) Veröffentlichungsnummer : **0 111 863**
**B1**

(12) # EUROPÄISCHE PATENTSCHRIFT

(45) Veröffentlichungstag der Patentschrift : **07.08.85**

(51) Int. Cl.⁴ : **C 07 C 49/80**, C 07 C 45/69

(21) Anmeldenummer : **83112470.6**

(22) Anmeldetag : **12.12.83**

(54) **Verfahren zur Herstellung von beta,4-Dichlor-propiophenon.**

(30) Priorität : **23.12.82 DE 3247823**

(43) Veröffentlichungstag der Anmeldung : **27.06.84 Patentblatt 84/26**

(45) Bekanntmachung des Hinweises auf die Patenterteilung : **07.08.85 Patentblatt 85/32**

(84) Benannte Vertragsstaaten : **DE FR GB IT**

(56) Entgegenhaltungen :
**DE-A- 1 768 624**
**DE-B- 2 851 371**
**DE-C- 733 694**
**CANADIAN JOURNAL OF RESEARCH, vol. VIII, 1933, publ. by the NATIONAL RESEARCH COUNCIL OF CANADA, C.F.H. ALLEN et al "The preparation of beta-chloropropiophenone", Seiten 440-446**

(73) Patentinhaber : **BAYER AG**
**Konzernverwaltung RP Patentabteilung**
**D-5090 Leverkusen 1 Bayerwerk (DE)**

(72) Erfinder : **Jautelat, Manfred, Dr.**
**Müllersbaum 28**
**D-5093 Burscheid (DE)**
Erfinder : **Hagedorn, Ferdinand, Dr.**
**Domblick 16**
**D-5090 Leverkusen 3 (DE)**
Erfinder : **Handschuh, Volkmar, Dr.**
**Luisenstrasse 19**
**D-5093 Burscheid (DE)**

**EP 0 111 863 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von β,4-Dichlor-propiophenon.

Es ist bereits bekannt, β,4-Dichlor-propiophenon durch eine Friedel-Crafts-Acylierung von Chlorbenzol und 3-Chlorpropionsäurechlorid herzustellen (J. Org. Chem. *12*, 96 (1947) ; Canad. J. Res. *8*, 440 (1933)). Dieses Verfahren hat jedoch den Nachteil, daß neben dem erwünschten 4-Chlor-Derivat auch das isomere 2-Chlor-Derivat entsteht, welches nur schwierig und unter großem Aufwand vom β,4-Dichlor-propiophenon abgetrennt werden kann.

Es ist ferner bekannt, β-Chlor-propiophenon durch Addition von Ethylen an Benzoylchlorid in Gegenwart von Aluminiumchlorid in Ethylbromid herzustellen (Canad. J. Res. *8*, 440 (1933)). In der selben Publikation werden 10 substituierte β-Chlorpropiophenone, u. a. auch β,4-Dichlor-propiophenon, beschrieben, die jedoch überraschenderweise nicht durch Addition von Ethylen an substituierte Benzoylchloride, sondern durch Friedel-Crafts-Acylierung von substituierten Benzolen und 3-Chlorpropionsäurechlorid hergestellt werden (Cand. J. Res. *8*, 440 (1933)). Die Übertragung der für Benzoylchlorid gemachten Vorschrift auf 4-Chlorbenzoylchlorid zur Herstellung von β,4-Dichlor-propiophenon ergab ein durch Oligomerisierung von Ethylen verunreinigtes Produkt, das sich nicht ausreichend reinigen ließ. Ein weiterer Nachteil des oben beschriebenen Verfahrens besteht in den erforderlichen Reaktionszeiten von 30 bis 35 Stunden für einen 0,4 Mol-Ansatz bei 20 °C.

Es wurde jetzt gefunden, daß man β,4-Dichlor-propiophenon in guter Ausbeute und hoher Reinheit durch Umsetzung von 4-Chlorbenzoylchlorid mit Ethylen unter Druck in einem Verdünnungsmittel in Gegenwart einer Lewis-Säure herstellen kann, wenn man die Umsetzung in Gegenwart von nicht mehr als der stöchiometrischen Menge Lewis-Säure (bezogen auf 4-Chlorbenzoylchlorid) im Temperaturbereich von 30 bis 100 °C und bei Drücken von 0,1 bis 100 bar durchführt.

Es ist als ausgesprochen überraschend zu bezeichnen, daß das Verfahren zur Herstellung von β-Chlorpropiophenon aus Benzoylchlorid und Ethylen (Canad. J. Res. *8*, 440 (1933)) sich nicht zur analogen Herstellung von β,4-Dichlor-propiophenon eignet. Wird statt des überschusses an Aluminiumchlorid als Katalysator nur die äquimolare Menge an Lewis-Säure (bezogen auf 4-Chlorbenzoylchlorid) verwendet, so verläuft die Addition von Ethylen an 4-Chlorbenzoylchlorid selektiv zum β,4-Dichlor-propiophenon. Es war ebenfalls nicht zu erwarten, daß die Steigerung der Temperatur auf Werte über 30 °C die Selektivität für die Ketonbildung, obgleich die thermische Unbeständigkeit dieser β-Chlorketone bekannt ist, erhöht. (« The β-chloroketone is easily decomposed by heat. » Canad. J. Res. *8*, 440, Zeile 21 (1933)).

Das erfindungsgemäße Verfahren weist eine Reihe von Vorteilen auf. So erhält man das β,4-Dichlor-propiophenon isomerenrein und kann auf eine aufwendige Trennung und Reinigung vom begleitenden β,2-Dichlor-propiophenon verzichten. Die Reaktionszeiten betragen nur wenige Stunden und die Ausbeuten sind nahezu quantitativ.

Der Reaktionsablauf kann durch das folgende Formel-schema wiedergegeben werden :

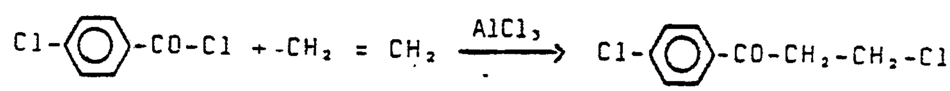


Die Ausgangsstoffe sind technisch zugängliche Produkte. Als Verdünnungsmittel kommen alle inerten organischen Lösungsmittel infrage. Hierzu gehören vorzugsweise halogenierte Kohlenwasserstoffe wie Dichlormethan, Chlorethan, 1,2-Dichlorethan, 1,2-Dichlorbenzol oder Trichlorbenzol ; Nitroalkane wie Nitromethan, Nitroethan oder Nitropropan ; Nitrobenzol sowie Schwefelkohlenstoff.

Als Katalysatoren kommen Lewis-Säuren in Frage, wie sie beispielsweise in Houben-Weyl, « Methoden der organischen Chemie », Band VII/2a, Georg Thieme Verlag Stuttgart 1973, auf Seite 22/23 in Tabelle 1 aufgeführt sind. Bevorzugt seien Aluminiumchlorid, Eisenchlorid, Zinkchlorid, Titantetrachlorid und Zinntetrachlorid genannt. Besonders bevorzugt wird Aluminiumchlorid als Lewis-Säure eingesetzt. Bedeutungsvoll ist die eingesetzte Menge an Lewis-Säure, die bezogen auf 4-Chlorbenzoylchlorid nur bis zur äquimolaren Menge verwendet wird.

Die Reaktionstemperaturen können im Bereich von 30 bis 100 °C, vorzugsweise 30 bis 70 °C, variiert werden.

Ethylen wird unter Druck eingeleitet oder aufgepreßt, wobei der Druck zwischen 0,1 und 100 bar, vorzugsweise 1 bis 20 bar, variiert wird.

Bei der Durchführung des erfindungsgemäßen Verfahrens setzt man die Reaktionspartner 4-Chlorbenzoylchlorid und Ethylen in einem Molverhältnis von 0,5 : 1 bis 2 : 1, vorzugsweise 1 : 1, um.

β,4-Dichlor-propiophenon dient als Ausgangsstoff zur Herstellung von optischen Aufhellern (DE-A-2 310 446).

## Herstellungsbeispiele

### Beispiel 1

26,7 g (0,2 mol) gepulvertes Aluminiumchlorid werden in 100 ml destilliertem Methylenchlorid im

Autoklaven (V4A) bei 25 °C unter $N_2$ vorgelegt. Nach Zugabe von 35,0 g (0,2 mol) p-Chlorbenzoylchlorid wird auf 50 °C erwärmt und 10 bar Ethylen aufgedrückt. Nach 3 Stungen ist Druckkonstanz erreicht. Die Reaktionslösung wird mit $CH_2Cl_2$ verdünnt und mit Eis sowie verdünnter Salzsäure aufgearbeitet. Nach dem Trocknen der Lösung über Natriumsulfat wird das Lösungsmittel im Vakuum abgezogen. Es bleiben 36,8 g (0,18 mol, 90 %) β,4-Dichlor-propiophenon als Öl zurück, das langsam durchkristallisiert (GC-Reinheit 98 %). Fp. 47 bis 49 °C (Beilstein VII/3, S. 1030 : Fp. 48 °C).

NMR ($CDCl_3$) ; δ 3,4 (t, J = 6,5 Hz, 2 H), 3,9 (t, J = 6,5 Hz, 2 H), 7,4 (d, J = 9 Hz, 2 H), 7,9 (d, J = 9 Hz, 2 H)

## Beispiel 2

176,5 g (1 Mol) 99,1 %iges 4-Chlorbenzoylchlorid werden zu einer Suspension von 134 g (0,975 Mol) 97 %igem Aluminiumchlorid wasserfrei in 180 g o-Dichlorbenzol (wasserfrei, andestillirt) unter Rühren und Feuchtigkeitsausschluß getropft, wobei sich das Gemisch auf 44 °C erwärmt. Durch Umsetzen dieses Gemisches mit gasförmigem Ethylen unter Rühren bei 50 °C unter einem Druck von 2 bar in einem Email-Autoklaven erhält man nach 6 Stunden und 45 Minuten ein Reaktionsgemisch, das beim Austragen in eine wäßrige Salzsäure (aus 600 ml Wasser und 100 ml konzentrierter Salzsäure) bei 50 °C hydrolysiert wird. Man trennt die Phasen, wäscht die organische Phase mit verdünnter Salzsäure, danach mit Soda-Lösung bei 25 bis 30 °C, ferner mit 150 ml Wasser. Die organische Phase (599 g) wird bis zu einer ca. 50 %igen Lösung andestilliert und zur Gehaltsbestimmung gegeben, Ausbeute 96,6 %.

## Beispiel 3

68,6 g (0,5 Mol) 97 %iges Aluminiumchlorid (gepulvert) werden in 200 ml wasserfreiem o-Dichlorbenzol bei 25 °C suspendiert. Zu dieser Mischung tropft man innerhalb 30 Minuten 89,5 g (0,5 Mol) 98 %iges 4-Chlorbenzoylchlorid unter Rühren und schwachem Kühlen. Die so hergestellte Lösung des Aluminiumchlorid-Komplexes wird in einem Einliter-Glasautoklaven unter Rühren auf 50 °C erwärmt und durch Aufdrücken von gasförmigem Ethylen bis zu einem Druck von maximal 6 bar innerhalb 2 1/4 Stunden umgesetzt. Man rührt das Gemisch nach 1,5 Stunden nach, entspannt den Druckbehälter und trägt den Inhalt auf ca. 600 g Eis aus, wobei man 10 ml konzentrierte Salzsäure zugibt. Man trennt die Phasen, wäscht die organische Phase zweimal mit je 50 ml Wasser, vereint die wäßrigen Phasen und schüttelt sie zweimal mit jeweils 50 ml o-Dichlorbenzol aus. Die vereinigten organischen Lösungen werden über eine einfache Destillationsbrücke im Wasserstrahlvakuum destilliert, bis eine etwa 50 %ige Lösung von β,4-Dichlor-propiophenon als Sumpf vorliegt. Ausbeute : 199,6 g Lösung mit einem Gehalt von 619 g/l ≙ 94 % d. Th.

**Patentansprüche**

1. Verfahren zur Herstellung von β,4-Dichlor-propiophenon durch Umsetzung von 4-Chlorbenzoylchlorid mit Ethylen unter Druck in einem Verdünnungsmittel in Gegenwart einer Lewis-Säure, dadurch gekennzeichnet, daß man die Umsetzung in Gegenwart von nicht mehr als der stöchiometrischen Menge Lewis-Säure (bezogen auf 4-Chlorbenzoylchlorid) im Temperaturbereich von 30 bis 100 °C und bei Drücken von 0,1 bis 100 bar durchführt.
2. Verfahren nach Anspruch 1, dadurch gekennzeichnet, daß man in Gegenwart stöchiometrischer Mengen Lewis-Säure arbeitet.
3. Verfahren nach Anspruch 1 und 2, dadurch gekennzeichnet, daß man die Umsetzung im Temperaturbereich von 30 bis 70 °C durchführt.
4. Verfahren nach Ansprüchen 1 bis 3, dadurch gekennzeichnet, daß man die Umsetzung bei Drücken von 1 bis 20 bar durchführt.
5. Verfahren nach Ansprüchen 1 bis 4, dadurch gekennzeichnet, daß man die Umsetzung in Gegenwart von Aluminiumchlorid durchführt.
6. Verfahren nach Ansprüchen 1 bis 5, dadurch gekennzeichnet, daß man 4-Chlorbenzoylchlorid mit Ethylen in einem Molverhältnis von 0,5 : 1 bis 2 : 1 umsetzt.

**Claims**

1. Process for the preparation of β,4-dichloropropiophenone by reaction of 4-chlorobenzoyl chloride with ethylene under pressure in a diluent in the presence of a Lewis acid, characterised in that the reaction is carried out in the presence of not more than the stoichiometric amount of Lewis acid (relative to 4-chlorobenzoyl chloride) in the temperature range from 30 to 100 °C and under pressures from 0.1 to 100 bar.
2. Process according to Claim 1, characterised in that it is carried out in the presence of stoichiometric amounts of Lewis acid.
3. Process according to Claims 1 and 2, characterised in that the reaction is carred out in the temperature range from 30 to 70 °C.

4. Process according to Claims 1 to 3, characterised in that the reaction is carried out under pressures from 1 to 20 bar.

5. Process according to Claims 1 to 4, characterised in that the reaction is carried out in the presence of aluminium chloride.

6. Process according to Claims 1 to 5, characterised in that 4-chlorobenzoyl chloride is reacted with ethylene in a molar ratio of 0,5 : 1 to 2 : 1.

**Revendications**

1. Procédé de production de β-4-dichloropropiophénone par réaction du chlorure de 4-chlorobenzoyle avec l'éthylène sous pression dans un diluant en présence d'un acide de Lewis, caractérisé en ce qu'on conduit la réaction en présence d'une quantité ne dépassant pas la quantité stœchiométrique d'acide de Lewis (par rapport au chlorure de 4-chlorobenzoyle) dans l'intervalle de température de 30 à 100 °C et à des pressions de 0,1 à 100 bars.

2. Procédé suivant la revendication 1, caractérisé en ce qu'on opère en présence de quantités stœchiométriques d'acide de Lewis.

3. Procédé suivant les revendications 1 et 2, caractérisé en ce qu'on conduit la réaction dans un intervalle de température de 30 à 70 °C.

4. Procédé suivant les revendications 1 à 3, caractérisé en ce qu'on conduit la réaction à des pressions de 1 à 20 bars.

5. Procédé suivant les revendications 1 à 4, caractérisé en ce qu'on conduit la réaction en présence de chlorure d'aluminium.

6. Procédé suivant les revendications 1 à 5, caractérisé en ce qu'on fait réagir du chlorure de 4-chlorobenzoyle avec de l'éthylène dans un rapport molaire de 0,5 : 1 à 2 : 1.